(19) Europäisches Patentamt
European Patent Office
Office européen des brevets

(11) EP 4 635 441 A1

(12) EUROPEAN PATENT APPLICATION
published in accordance with Art. 153(4) EPC

(43) Date of publication:
22.10.2025 Bulletin 2025/43

(21) Application number: 23903907.6

(22) Date of filing: 08.12.2023

(51) International Patent Classification (IPC):
A61B 34/10 (2016.01)        A61B 34/30 (2016.01)
B25J 9/16 (2006.01)

(52) Cooperative Patent Classification (CPC):
A61B 34/10; A61B 34/30; B25J 9/16

(86) International application number:
PCT/KR2023/020248

(87) International publication number:
WO 2024/128704 (20.06.2024 Gazette 2024/25)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 12.12.2022 KR 20220173116

(71) Applicant: Curexo, Inc.
Seoul 05814 (KR)

(72) Inventors:
• KIM, Bong Oh
  Seoul 05814 (KR)
• KIM, Soo Jong
  Seoul 05814 (KR)
• LIM, Heung Soon
  Seoul 05814 (KR)

(74) Representative: Stöckeler, Ferdinand et al
Schoppe, Zimmermann, Stöckeler
Zinkler, Schenk & Partner mbB
Patentanwälte
Radlkoferstrasse 2
81373 München (DE)

(54) **APPARATUS FOR GENERATING OPERABLE AREA OF SURGICAL ROBOT AND METHOD THEREOF**

(57) Disclosed are an apparatus and method for generating an operable area of a surgical robot, the method of generating the operable area according to the disclosure including the steps of setting virtual locations of an affected area and a surgical robot and a distance between the affected area and the surgical robot; determining the location, shape, size of an operable area based on the virtual locations and the distance; determining a plurality of test positions for simulating movement of an end effector of a surgical robot in an interior or on a surface of the operable area; simulating the movement of the end effector according to a previously planned cutting path of the surgical robot, for each test position; and generating a final operable area based on simulation results at the plurality of test positions. With this, an operable area where no problem occurs in deriving a kinematic solution of a surgical robot is identified before surgery, thereby effectively preventing interruption or delay in a surgical procedure.

[Fig.2]

Start

Set virtual locations and distance of affected area and surgical robot — S100

Determine location, shape and size of initial operable area — S110

Determine test positions in interior or on surface of initial operable area — S120

Simulate movement of surgical robot according to cutting path for each test position — S130

S140 — Do simulations at all test positions result in pass?  YES

NO

Modify initial operable area — S150

Determine final operable area — S160

End

## Description

[TECHNICAL FIELD]

The disclosure relates to an apparatus and method for generating an operable area of a surgical robot, and more particularly to an apparatus and method for generating an operable area where no problem occurs in deriving a kinematic solution of a surgical robot during a surgical operation.

[BACKGROUND ART]

For general industrial robots, teaching is performed to make the robots remember an operation sequence, location, pose, etc. of a manipulator according to task details before performing an actual task using the robot. The teaching is carried out in such a way that a worker teaches the movements by holding the tip of the manipulator in person or by using a separate operation button. Regarding some areas where the teaching has not been performed, image information is used to define the location, pose, etc. of the manipulator in real time, but this is applicable only to extremely limited areas. Most tasks of industrial robots are performed based on the previously performed teaching.

In an industrial environment, the teaching is possible because the location or movement in a work process does not deviate significantly from a set routine. On the other hand, in a surgical environment, it is virtually impossible for a doctor to teach the surgical robot the movements based on a cutting path in advance because the location, pose, etc. of a patient may change depending on situations.

In particular, in the case of active robotic surgery, the robot automatically performs surgery according to a planned cutting path without the intervention of a surgeon, and the robot moves along a cutting path defined based on an implant coordinate system. As described above, it is difficult to teach the surgical robot before surgery, and thus a kinematic solution may not be derived during the movement of the robot depending on the relative location and pose of the implant coordinate system with respect to the origin of the surgical robot. If such a problem occurs during the surgery, the surgery is delayed because it is required to move the location of the patient or robot, or modify the planned cutting path while the surgery is stopped.

Accordingly, if an operable area where no problem occurs in deriving the kinematic solution of the surgical robot is identified in advance, it is expected that the surgical process is carried out efficiently.

[DISCLOSURE]

[TECHNICAL PROBLEM]

The disclosure has been conceived to solve the problems of the related art as mentioned above, and an aspect of the disclosure is to provide an apparatus and method for generating an operable area of a surgical robot, which generate and provide the operable area where no problem occurs in deriving a kinematic solution of a surgical robot.

[TECHNICAL SOLUTION]

According to an embodiment of the disclosure, the foregoing aspect is achieved by a method of generating an operable area of a surgical robot, in which steps are performed by a processor, the method including the steps of: (a) setting virtual locations of an affected area and a surgical robot and a distance between the affected area and the surgical robot; (b) determining the location, shape, size of an operable area based on the virtual locations and the distance; (c) determining a plurality of test positions for simulating movement of an end effector of a surgical robot in an interior or on a surface of the operable area; (d) simulating the movement of the end effector according to a previously planned cutting path of the surgical robot, for each test position; and (e) generating a final operable area based on simulation results at the plurality of test positions.

In this case, the cutting path of the surgical robot may be defined based on a preset reference coordinate system, and the step of simulating the movement of the end effector may include simulating the movement of the end effector according to the cutting path with respect to multiple poses of the reference coordinate system, by performing simulation while locating the origin of the reference coordinate system at each test position and changing the pose of the reference coordinate system corresponding to a preset pose changing range.

Here, the method may further include steps of: modifying at least one among the location, size and shape of the operable area based on the simulation results at each test position; and re-performing the step (c) and the step (d) with respect to the modified operable area.

Further, the method may further include steps of: modifying the cutting path based on the simulation results at each test position; and re-performing the simulation using the modified cutting path.

Meanwhile, the locations and number of test positions may be determined based on the shape and size of the operable area.

In addition, the operable area may be shaped like a sphere, a cylinder, or a polygonal prism.

According to another embodiment of the disclosure, the foregoing aspect is achieved by an apparatus for generating an operable area to set the operable area of a surgical robot, the apparatus including a processor, the processor being configured to: set virtual locations of an affected area and the surgical robot and a distance between the affected area and the surgical robot, determine the location, shape, size of an operable area based on the virtual locations and the distance, determine a plurality of test positions for simulating movement of an end effector of the surgical robot in an interior or on a surface of the operable area, simulate the movement of the end effector according to a previously planned cutting path of the surgical robot, for each test position, and generate a final operable area based on simulation results at the plurality of test positions.

Here, the cutting path of the surgical robot may be planned based on a preset reference coordinate system, and the processor may simulate the movement of the end effector according to the cutting path with respect to multiple poses of the reference coordinate system, by performing simulation while locating the origin of the reference coordinate system at each test position and changing the pose of the reference coordinate system corresponding to a preset pose changing range.

Meanwhile, the processor may be configured to modify at least one among the location, size and shape of the operable area based on the simulation results at each test position, and re-simulate the movement of the end effector with respect to the modified operable area.

In addition, the processor may be configured to modify the cutting path based on the simulation results at each test position, and re-simulate the movement of the end effector using the modified cutting path.

[ADVANTAGEOUS EFFECTS]

As described above, according to the disclosure, an operable area where no problem occurs in deriving a kinematic solution of a surgical robot is identified before surgery, thereby effectively preventing interruption or delay in a surgical procedure.

[DESCRIPTION OF DRAWINGS]

FIG. 1 is a block diagram schematically showing the configuration of an apparatus for generating an operable area of a surgical robot according to an embodiment of the disclosure;
FIG. 2 is a flowchart showing a method of generating an operable area of a surgical robot according to a first embodiment of the disclosure;
FIG. 3 is a reference diagram for describing how an apparatus for generating an operable area according to an embodiment of the disclosure determines a simulation test position;
FIG. 4 is a reference diagram for describing how an apparatus for generating an operable area according to an embodiment of the disclosure simulates the movement of a surgical robot based on a cutting path at each test position; and
FIG. 5 is a flowchart showing a method of generating an operable area by an apparatus for generating the operable area according to a second embodiment of the disclosure.

[BEST MODE]

Below, specific embodiments of the disclosure will be described with reference to the drawings. However, detailed descriptions of known functions or configurations that may obscure the gist of the disclosure in the following description and the accompanying drawings will be omitted. Further, it should be noted that like numerals refer to like elements identical components are indicated with the same drawing reference numerals throughout the drawings.

A surgical robot set forth herein refers to a medical robot that performs cutting a surgical target bone in order to install an implant during artificial joint replacement surgery. In this case, the artificial joint replacement surgery includes total knee replacement surgery, partial knee replacement surgery, and hip joint replacement surgery.

An apparatus for generating an operable area of a surgical robot according to the disclosure simulates the movement of the surgical robot to generate the operable area in which the surgical robot can safely perform surgery. Here, the operable area refers to an area in which a problem in deriving a kinematic solution (joint angle) of the surgical robot, such as a singularity, does not occur when an end effector, i.e. a surgical tool attached to the end of a robot arm of the surgical robot performs cutting while moving.

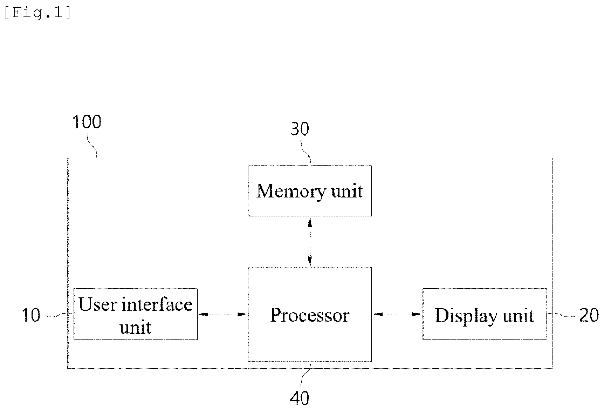

FIG. 1 is a block diagram schematically showing the configuration of an apparatus 100 for generating the operable area of a surgical robot according to an embodiment of the disclosure.

Referring to FIG. 1, an apparatus 100 for generating the operable area includes a user interface unit 10, a display unit 20, a memory unit 30, and a processor 40.

The user interface unit 10 refers to a module for receiving various inputs from a user in order to generate the operable area of the surgical robot, and may be implemented by various input devices such as a mouse, a keyboard, a keypad, and a button.

The display unit 20 is to display various pieces of information including an image, a graphic, etc. on a screen, and may be implemented by a liquid crystal display (LCD) panel, a light emitting diode (LED) panel, an organic light emitting diode (OLED) panel, etc. Further, the user input unit 10 and the display unit 20 may be integrated and implemented as a single device like a touch screen. The display unit 20 displays various graphic user interfaces (GUI) and processing outputs provided in the process of deriving the operable area by simulating the movement of the surgical robot according to the cutting path.

The memory unit 30 is implemented by memory elements such as a random access memory (RAM), a flash memory, and an erasable and programmable read only memory (EPROM), and may store various operating systems (OS), middleware, platforms, and various applications for the apparatus 100 for generating the operable area and may store program codes and signal-processed image signals, voice signals, and various data.

Further, the memory unit 30 stores a surgical robot simulation model designed to have the same motion characteristics as an actual surgical robot's manipulator, etc., and a motion planning algorithm applied to the actual surgical robot, in order to simulate the movement of the end effector of the surgical robot according to the cutting path. For reference, the robot motion planning algorithm refers to an algorithm that generates the movements of a robot's manipulator, etc. to move from an arbitrary start point to an arbitrary end point when that start point and that end point are given to the robot, and may employ known motion planning algorithms such as a criticality-based algorithm, a sampling-based algorithm, a decomposition-based algorithm, or a potential fields-based algorithm.

Meanwhile, the memory unit 30 may store the cutting path of the surgical robot, which has been planned in advance. The cutting path includes information about the locations of the surgical tool to be moved when cutting is performed by the surgical robot and the pose (angle and direction) of the surgical tool at those locations. The cutting path may be generated as information about the locations of the surgical tool during the cutting process and the pose of the surgical tool at those locations based on a preset reference coordinate system. Here, the reference coordinate system may employ an implant coordinate system defined for an implant to be installed in an affected area after the cutting is performed by the surgical robot.

The processor 40 uses user input information input through the user interface unit 10 and the surgical robot simulation model, the motion planning algorithm, etc. stored in the memory unit 30 to simulate the movement of the end effector of the surgical robot according to the cutting path planned in advance, thereby generating the operable area of the surgical robot.

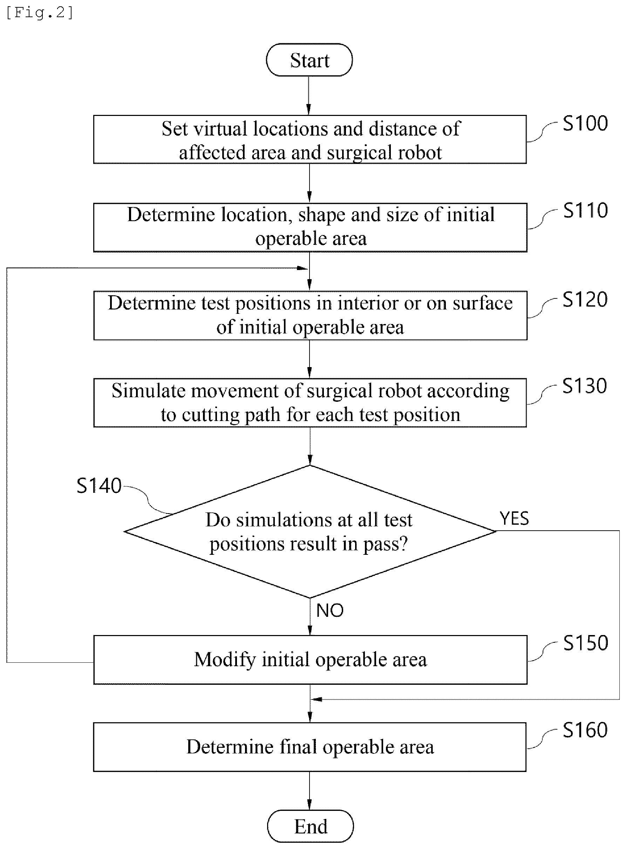

Below, a method of generating an operable area by the apparatus 100 for generating the operable area will be described with reference to FIG. 2.

FIG. 2 is a flowchart showing a method of generating an operable area of a surgical robot according to a first embodiment of the disclosure.

Referring to FIG. 2, the processor 40 sets virtual locations of an affected area to be operated and a surgical robot, and a distance between the affected area and the surgical robot (S100). The virtual locations may be determined as absolute locations at which the affected area and the surgical robot are to be placed in a virtual surgical space simulating an actual physical surgical space, or, alternatively, the virtual location of the affected area may be determined as a relative location at which the affected area is to be placed with respect to the surgical robot placed at a predetermined location. For example, the relative location of the affected area may be defined as a location at which the affected area is spaced apart from the surgical robot by predetermined distances in X-, Y- and Z-axial directions, respectively.

The virtual locations of the affected area and the surgical robot may be set based on a user input using the user interface unit 10, or may be set based on placement reference information about the surgical robot and the affected area according to the types of surgery, which has been stored in the memory unit 30 in advance.

Meanwhile, the distance between the affected area and the surgical robot may be expressed as distances by which the affected area is spaced apart from the surgical robot in the X-, Y- and Z-axial directions, based on the virtual locations of the affected area and the surgical robot.

Next, the processor 40 determines the location, shape and size of an initial operable area based on the virtual locations of the affected area and the surgical robot and the distance between the affected area and the surgical robot determined in the previous step (S110). Here, the initial operable area refers to an area set before the simulation, and the above area becomes a simulation target area. In other words, the simulation of the surgical robot according to the cutting path is carried out within the initial operable area, and the simulation is not performed for areas beyond this area. The initial operable area is different in meaning from a final operable area finally determined based on simulation results after the simulation.

The initial operable area may be determined as a predetermined three-dimensional shape including the location of the affected area set in step S100, and the size and location of the initial operation area may be determined in consideration of a robot arm length of the surgical robot, the kinematic characteristics of the surgical robot, a relative location and distance between the surgical robot and the affected area. Further, the initial operable area and the final operable area, which is finally produced by the processor 40 as will be described below, may be generated as various three-dimensional shapes, such as a sphere, a cylinder, a cube, a rectangular parallelepiped, and a polygonal prism, but are not particularly limited to the shape.

The memory unit 30 may store reference information to determine the location, shape and size of the operable area based on the length of the robot arm, the kinematic characteristics of the surgical robot, the type of surgery, the relative location and distance between the affected area and the surgical robot, and may determine the location, shape and size of the operable area suitable for the location and distance set in step S100 based on that reference information.

The operable area is defined based on the surgical robot coordinate system because it is an area where stable motion control of the surgical robot is possible without the occurrence of the singularity when the cutting is performed by the surgical robot. For reference, the surgical robot coordinate system refers to a coordinate system defined with the origin of a point on the surgical robot, for example, a point on a robot base, and the surgical robot is actually controlled based on the surgical robot coordinate system.

Further, the operable area may be determined as a single area, but may also be determined as multiple areas that are separate from each other or at least partially overlapping with each other.

Next, the processor 40 determines a plurality of test positions for simulating the movement of the end effector of the surgical robot in the interior or on the surface of the initial operable area determined in the previous step (S120). Here, the surface may also be referred to as the boundary of the area.

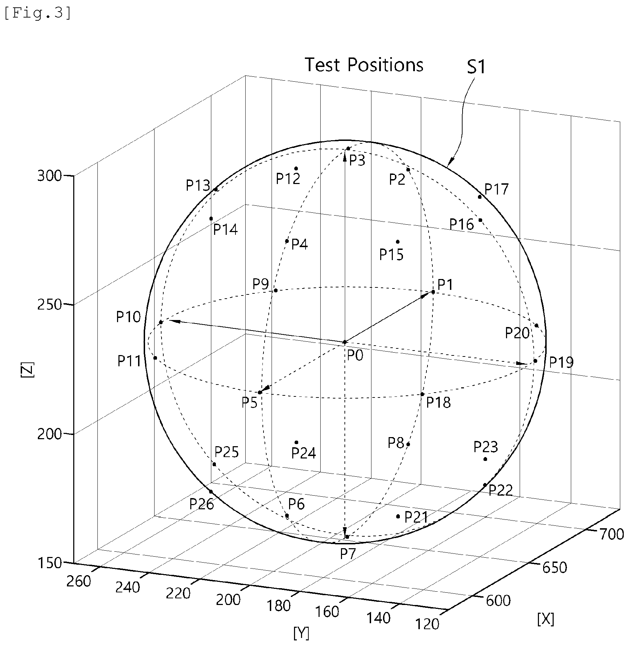

FIG. 3 is a reference diagram for describing how the apparatus 100 for generating the operable area according to an embodiment of the disclosure determines the simulation test positions, and illustrates an example that the test positions are determined for the initial operable area having a spherical shape.

The simulation test position is determined as one or more points in the interior or on the surface of the initial operable area. FIG. 3 shows an example that multiple points at the inner center of the initial operable area S1 having a spherical shape and on the surface of the initial operable area S1 are determined as the test positions P0 to P26.

The locations and number of the test positions P may be vary depending on the shape and size of the initial operable area S1. For example, when the initial operable area S1 is relatively large, the number of test positions P may increase relatively compared to that of when the initial operable area S1 is small. Meanwhile, the distance between the adjacent test positions P may vary depending on the specific shape of the initial operable area S1.

The plurality of test positions P may be determined as locations spaced apart by a predetermined distance on the surface of the initial operable area S1. In this case, the distance between the test positions P may be set differently according to the distance between the corresponding point of the initial operable area S1 and the surgical robot, e.g., the distance from the origin of the surgical robot on the robot base. Accordingly, even for the points on the surface of the initial operable area S1, the distance from the adjacent test position P may be set differently according to the specific distance from the surgical robot.

The memory unit 30 may store reference information to determine the simulation test position for the operable area, such as distance information between the test positions according to the shape and size of the operable area, the distance between the point in the interior or on the surface of the operable area and the surgical robot, and the processor 40 may determine the number and locations of test positions based on the reference information.

As described above, when the simulation test positions are determined, the processor 40 simulates the movement of the surgical robot according to the planned cutting path of the surgical robot for each test position (S130).

For reference, the cutting path needs to be planned only before the simulation, and thus the planning of the cutting path may be done at any point in time as long as it is before the simulation. For example, the planning of the cutting path may be done before the processor 40 determines the size, location, etc. of the initial operable area, or after the processor 40 determines the test positions. As described above, the cutting path is planned as the information about the locations of the surgical tool and the poses of the surgical tool at those locations during the cutting process based on the implant coordinate system, and is then stored in the memory unit 30.

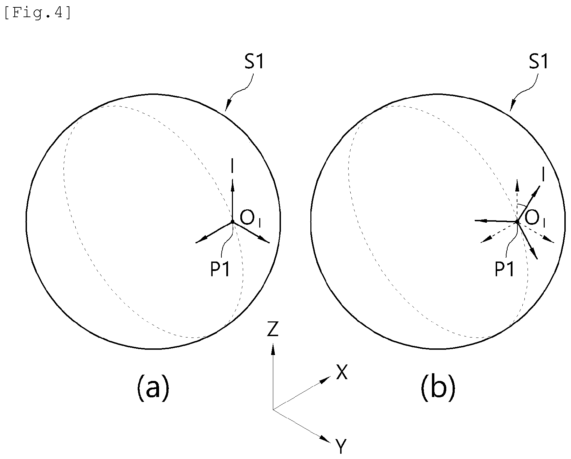

FIG. 4 is a reference diagram for describing how the apparatus 100 for generating the operable area according to an embodiment of the disclosure simulates the movement of a surgical robot based on a cutting path at each test position, and shows an example of performing a simulation at one test position among the plurality of test positions.

As shown in (a) of FIG. 4, the processor 40 locations the origin $O_I$ of the reference coordinate system, i.e., the implant coordinate system I, which is used as a reference for planning the cutting path, at one test position P1 among the plurality of test positions. In this way, as the location and pose of the implant coordinate system are determined, the processor 40 may calculate a transformation matrix $T_{Implant}^{Robot}$ between the robot coordinate system and the implant coordinate system.

Because a transformation matrix $T_{Cutting\ path\ point}^{Implant}$ between the implant coordinate system and the location of the surgical tool on the cutting path is derived during the planning of the cutting path, the processor 40 may simulate the movement of the surgical tool according to the cutting path at the simulation test position by applying the motion planning algorithm corresponding to the surgical robot.

However, even if it is assumed that the implant coordinate system is located at the test position in an actual surgical environment, it is difficult to specify a direction or pose where the affected area is located relative to the surgical robot, and the direction or pose may change during the surgical process. Therefore, it is required to perform simulations according to the multiple poses of the implant coordinate system while changing the pose of the implant coordinate system at the test position.

Accordingly, as shown in (b) of FIG. 4, the pose (angle) of the implant coordinate system I may be changed by rotating the implant coordinate system I while the location of the origin $O_I$ of the implant coordinate system I is maintained at the test position P1. The processor 40 updates the transformation matrix $T^{Robot}_{Implant}$ between the robot coordinate system and the implant coordinate system in response to the pose change of the implant coordinate system, and may re-simulate the movement of the surgical tool along the cutting path for the changed pose of the implant coordinate system.

A changing range or interval for the pose of the implant coordinate system may be set based on preset standards or an input using the user input unit 10. The processor 40 may simulate the movement of the surgical tool according to the cutting path while changing the pose of the implant coordinate system at the test position P1 based on the changing range and interval set as above.

The processor 40 repeats a series of processes of 'locating the origin of the implant coordinate system at the corresponding test position → simulating the movement of the surgical tool along the cutting path → changing the pose of the implant coordinate system at the corresponding test position → re-simulating the movement of the surgical tool according to the cutting path for the changed pose' for each of the plurality of test positions determined in step S120, as shown in FIG. 4, thereby simulating the movement of the surgical tool according to the cutting path corresponding to the multiple poses of the implant coordinate system at each test position.

The simulation may result in a pass or a fail for each test position. When a problem occurs in deriving a kinematic solution, e.g., a singularity occurs during the simulation according to the cutting path at the corresponding test position, the simulation may result in the fail. On the other hand, when all cutting tasks are completed along the cutting path without any problem described above, the simulation may result in the pass. Meanwhile, the simulation may result in the fail even when the simulation results in the fail for some of the multiple poses of the implant coordinate system at the corresponding test position. When the simulation results in the fail for only some of the poses as described above, information may be provided regarding how many poses the simulation results in the pass or the fail for.

The simulation results may be displayed on the display unit 20 in the form of text corresponding to preset identification (ID) for each test position, or may be provided as visualized with the test positions different in color, etc. according to the simulation results at the corresponding test positions on graphics indicating the operable area corresponding to a simulation target range and the locations of the test positions.

When the simulations are completed for all the test positions through the foregoing process, subsequent steps are performed to generate the final operable area based on the simulation results.

In other words, when all the simulations result in the pass for all the test positions, the initial operable area, i.e., the target area for which the simulations have been previously performed, may be determined as the final operable area (S140). On the other hand, when the cutting task simulations result in the fail for some or all of the test positions, at least one among the location, size and shape of the previously generated operable area is modified (S150), and then step S120 of determining a plurality of test positions for the modified operable area and step S130 of simulating the movement of the surgical tool according to the planned cutting path while locating the implant coordinate system, used as the reference for planning the cutting path, at the determined test positions, and changing the poses are performed again.

The foregoing process is repeated until all the cutting task simulations of the surgical tool according to the multiple poses of the implant coordinate system are successfully completed for all the test positions of the modified operable area, and the operable area, in which all the simulations according to the multiple poses of the implant coordinate system result in the pass at all the test positions, is finally determined as the final operable area (S160).

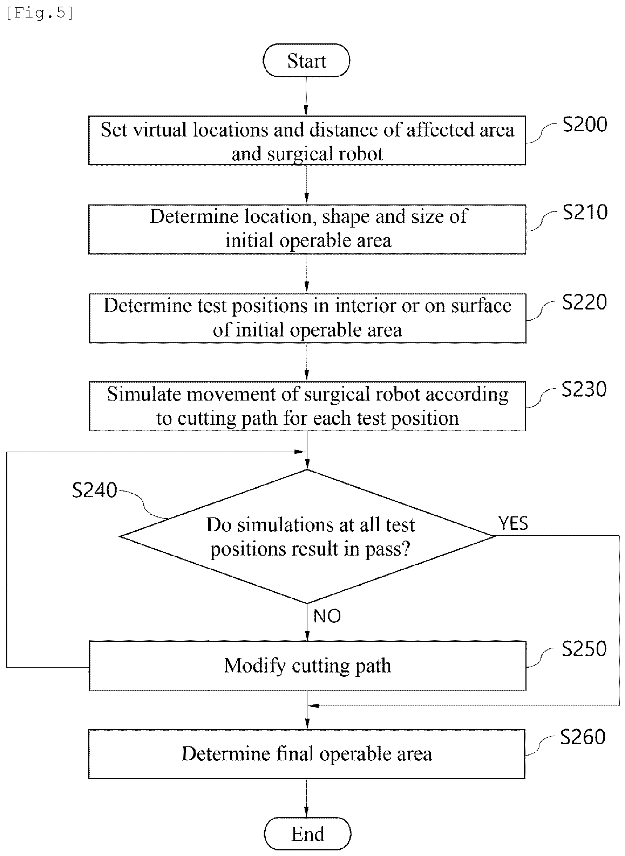

FIG. 5 is a flowchart showing a method of generating an operable area by the apparatus 100 for generating the operable area according to a second embodiment of the disclosure. To avoid repetitive descriptions, the same details as those of the foregoing embodiments will be omitted.

Referring to FIG. 5, the processor 40 sets virtual locations of an affected area to be operated and the surgical robot, and a distance between the affected area and the surgical robot (S200), determines the location, shape and size of the initial operable area based on the determined virtual locations of the affected area and the surgical robot and the determined distance between the affected area and the surgical robot (S210), determines a plurality of test positions for simulating the movement of the end effector of the surgical robot in the interior or on the surface of the initial operable area determined in step S210 (S220), and simulates the movement of the surgical robot according to the cutting path with respect to the plurality of poses of the implant coordinate system by performing the simulation while locating the implant coordinate system at each test position and changing the pose of the implant coordinate system (S230), the steps of which are the same as those of the first embodiment described with reference to FIG. 2.

However, when the cutting task simulations result in the fail with respect to all or some poses of the implant coordinate system at all or some test positions, the modification is made to the operable area corresponding to the simulation target area range according to the first embodiment. On the other hand, the second embodiment is different from the first embodiment in that the modification is made to the planned cutting path.

In other words, when all the simulations result in the pass at all the test positions, the processor 40 may immediately determine the operable area, i.e., the target area where the simulation have been performed, as the final operable area (S240, S260). On the other hand, when the cutting task simulations result in the fail with respect to some or all of the test positions, the processor 40 modifies the previously planned cutting path (S250), and re-performs step S230 of simulating the movement of the surgical tool based on the modified cutting path for each previously determined test position.

The modification of the cutting path may be carried by modifying the locations of the surgical tool at cutting locations forming the cutting path or the poses of the surgical tool at the corresponding locations. In this case, both the location and pose of the surgical tool may be modified, or only one of the location and the pose may be modified.

As a result of performing the simulation again using the modified cutting path, when all the simulations according to the multiple poses of the implant coordinate system at all the test positions result in the pass, the operable area applied to the simulation is determined as the final operable area (S260). When the simulation results in fail even for the modified cutting path, the process of modifying the cutting path again and performing the simulation based on the modified cutting path is repeated.

The generated final operable area may be provided through the display unit 20 as visualized in a graphic form having the location, shape and size of the operable area relative to the location of the surgical robot.

The steps of the foregoing methods described with reference to FIGS. 2 and 5 may be applied as modified or added according to the situations.

In the foregoing description, the operable area or the cutting path was alternatively modified according to the simulation results, but of course, both of them may be modified at once.

As described above, in the apparatus and method for generating the operable area of the surgical robot according to the disclosure, the operable area where no problem occurs in deriving a kinematic solution of the surgical robot may be identified in advance before surgery, thereby effectively preventing interruption or delay in a surgical procedure.

Although all elements for an embodiment of the disclosure are combined as one or operating as combined, the disclosure is not limited to this embodiment. In other words, one or more elements among all the elements may be selectively combined and operated as long as they fall into the scope of the disclosure. Further, all the elements may be respectively implemented by independent pieces of hardware, or some or all of the elements may be selectively combined and thus implemented as a computer program having a program module that performs the functions of some or all combinations from one or multiple pieces of hardware. Codes and code segments constituting that computer program may be easily deduced by those skilled in the art. Such a computer program is stored in a computer readable medium and read and executed by a computer, thereby implementing the embodiments of the disclosure. The storage medium of the computer program may include a magnetic recording medium, an optical recording medium, etc.

Further, the terms "comprise," "configure" and/or "have" specify the presence of stated components, unless there is a clearly different meaning in the disclosure, but do not preclude the presence thereof and should be construed to further include other components. Unless otherwise defined, all terms including technical or scientific terms used herein have the same meaning as commonly understood by those skilled in the art to which the disclosure pertains. General terms that are defined in a dictionary shall be construed to have meanings that are consistent in the context of the relevant art, and will not be interpreted as having an idealistic or excessively formalistic meaning unless clearly defined in the disclosure.

The foregoing description is merely an example of technical idea according to the disclosure, and various modifications and changes may be made by a person having ordinary knowledge in the art to which the disclosure pertains without departing from the essential characteristics of the disclosure. Accordingly, the embodiments of the disclosure are not intended to limit but rather to describe the technical idea of the disclosure, and the scope of the technical idea of the disclosure is not limited by these embodiments. The scope of the disclosure should be interpreted based on the appended claims, and all technical ideas within the equivalent scope should be interpreted as falling into the scope of the disclosure.

## Claims

1. A method of generating an operable area of a surgical robot, in which steps are performed by a processor, the method comprising the steps of:

   (a) setting virtual locations of an affected area and a surgical robot and a distance between the affected area and the surgical robot;
   (b) determining the location, shape, size of an operable area based on the virtual locations and the distance;
   (c) determining a plurality of test positions for simulating movement of an end effector of a surgical robot in an interior or on a surface of the operable area;
   (d) simulating the movement of the end effector according to a previously planned cutting path of the surgical robot, for each test position; and
   (e) generating a final operable area based on simulation results at the plurality of test positions.

2. The method of claim 1, wherein

   the cutting path of the surgical robot is defined based on a preset reference coordinate system, and

the step of simulating the movement of the end effector comprises simulating the movement of the end effector according to the cutting path with respect to multiple poses of the reference coordinate system, by performing simulation while locating the origin of the reference coordinate system at each test position and changing the pose of the reference coordinate system corresponding to a preset pose changing range.

3. The method of claim 1, further comprising steps of:

modifying at least one among the location, size and shape of the operable area based on the simulation results at each test position; and re-performing the step (c) and the step (d) with respect to the modified operable area.

4. The method of claim 1, further comprising steps of:

modifying the cutting path based on the simulation results at each test position; and re-performing the simulation using the modified cutting path.

5. The method of claim 1, wherein the locations and number of test positions are determined based on the shape and size of the operable area.

6. The method of claim 1, wherein the operable area is shaped like a sphere, a cylinder, or a polygonal prism.

7. An apparatus for generating an operable area to set the operable area of a surgical robot, the apparatus comprising a processor, the processor being configured to:

set virtual locations of an affected area and the surgical robot and a distance between the affected area and the surgical robot, determine the location, shape, size of an operable area based on the virtual locations and the distance, determine a plurality of test positions for simulating movement of an end effector of the surgical robot in an interior or on a surface of the operable area, simulate the movement of the end effector according to a previously planned cutting path of the surgical robot, for each test position, and generate a final operable area based on simulation results at the plurality of test positions.

8. The apparatus of claim 7, wherein

the cutting path of the surgical robot is planned based on a preset reference coordinate system, and
the processor simulates the movement of the end effector according to the cutting path with respect to multiple poses of the reference coordinate system, by performing simulation while locating the origin of the reference coordinate system at each test position and changing the pose of the reference coordinate system corresponding to a preset pose changing range.

9. The apparatus of claim 7, wherein the processor is configured to modify at least one among the location, size and shape of the operable area based on the simulation results at each test position, and re-simulate the movement of the end effector with respect to the modified operable area.

10. The apparatus of claim 7, wherein the processor is configured to modify the cutting path based on the simulation results at each test position, and re-simulate the movement of the end effector using the modified cutting path.

[Fig.1]

100
30

Memory unit

User interface unit — 10

Processor

Display unit — 20

40

[Fig.2]

Start

Set virtual locations and distance of
affected area and surgical robot — S100

Determine location, shape and size of initial
operable area — S110

Determine test positions in interior or on surface
of initial operable area — S120

Simulate movement of surgical robot according
to cutting path for each test position — S130

S140 — Do simulations at all test
positions result in pass? — YES

NO

Modify initial operable area — S150

Determine final operable area — S160

End

[Fig.3]

Test Positions

[Fig.4]

(a)                    (b)

[Fig.5]

```
                          ┌─────────┐
                          │  Start  │
                          └────┬────┘
                               │
                               ▼
        ┌──────────────────────────────────────────┐
        │ Set virtual locations and distance of      │ ⌐ S200
        │ affected area and surgical robot            │
        └──────────────────────┬─────────────────────┘
                               │
                               ▼
        ┌──────────────────────────────────────────┐
        │ Determine location, shape and size of      │ ⌐ S210
        │ initial operable area                       │
        └──────────────────────┬─────────────────────┘
                               │
                               ▼
        ┌──────────────────────────────────────────┐
        │ Determine test positions in interior or on │ ⌐ S220
        │ surface of initial operable area            │
        └──────────────────────┬─────────────────────┘
                               │
                               ▼
        ┌──────────────────────────────────────────┐
        │ Simulate movement of surgical robot         │ ⌐ S230
        │ according to cutting path for each test     │
        │ position                                    │
        └──────────────────────┬─────────────────────┘
                               │
                               ▼
              S240 ⌐      ◇ Do simulations at all test    ◇  YES
                         ◇ positions result in pass?       ◇──────┐
                               │ NO                               │
                               ▼                                  │
        ┌──────────────────────────────────────────┐              │
        │ Modify cutting path                         │ ⌐ S250     │
        └──────────────────────┬─────────────────────┘              │
                               │◄─────────────────────────────────┘
                               ▼
        ┌──────────────────────────────────────────┐
        │ Determine final operable area               │ ⌐ S260
        └──────────────────────┬─────────────────────┘
                               │
                               ▼
                          ┌─────────┐
                          │   End   │
                          └─────────┘
```

## INTERNATIONAL SEARCH REPORT

| International application No. |
|---|
| **PCT/KR2023/020248** |

| A. | CLASSIFICATION OF SUBJECT MATTER |
|---|---|

**A61B 34/10**(2016.01)i; **A61B 34/30**(2016.01)i; **B25J 9/16**(2006.01)i

According to International Patent Classification (IPC) or to both national classification and IPC

| B. | FIELDS SEARCHED |
|---|---|

Minimum documentation searched (classification system followed by classification symbols)

A61B 34/10(2016.01); A61B 17/00(2006.01); A61B 19/00(2006.01); A61B 34/00(2016.01); A61B 34/20(2016.01); A61B 34/30(2016.01); G06N 99/00(2010.01); G06T 1/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수술 로봇(surgical robot), 수술 가능 영역(surgical enable zone), 시뮬레이션(simulation), 가이드(guide), 위치(position), 자세(pose), 절삭 경로(cutting path)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2274167 B1 (CUREXO INC.) 12 July 2021 (2021-07-12) See claims 8 and 12; and paragraph [0073]. | 1,3-7,9-10 |
| A | | 2,8 |
| Y | JP 2008-173159 A (HITACHI MEDICAL CORP.) 31 July 2008 (2008-07-31) See abstract; and paragraph [0110]. | 1,3-7,9-10 |
| A | KR 10-2010-0112310 A (WOORIDUL HOSPITAL) 19 October 2010 (2010-10-19) See entire document. | 1-10 |
| A | KR 10-2019-0080703 A (HUTOM CO., LTD.) 08 July 2019 (2019-07-08) See entire document. | 1-10 |
| A | KR 10-2022-0158737 A (MAKO SURGICAL CORP.) 01 December 2022 (2022-12-01) See entire document. | 1-10 |

☐ Further documents are listed in the continuation of Box C.  ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 April 2024** | **09 April 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/020248**

| Patent document cited in search report | | | Publication date (day/month/year) | Patent family member(s) | | | Publication date (day/month/year) |
|---|---|---|---|---|---|---|---|
| KR | 10-2274167 | B1 | 12 July 2021 | EP | 4026509 | A2 | 13 July 2022 |
| | | | | EP | 4026509 | A4 | 30 August 2023 |
| | | | | JP | 2022-545743 | A | 28 October 2022 |
| | | | | JP | 7341567 | B2 | 11 September 2023 |
| | | | | KR | 10-2021-0029322 | A | 16 March 2021 |
| | | | | US | 11666392 | B2 | 06 June 2023 |
| | | | | US | 2022-0265364 | A1 | 25 August 2022 |
| | | | | WO | 2021-045546 | A2 | 11 March 2021 |
| | | | | WO | 2021-045546 | A3 | 29 April 2021 |
| JP | 2008-173159 | A | 31 July 2008 | JP | 4762160 | B2 | 31 August 2011 |
| KR | 10-2010-0112310 | A | 19 October 2010 | KR | 10-1057702 | B1 | 18 August 2011 |
| KR | 10-2019-0080703 | A | 08 July 2019 | KR | 10-1880246 | B1 | 19 July 2018 |
| | | | | KR | 10-2019-0080702 | A | 08 July 2019 |
| | | | | KR | 10-2019-0088375 | A | 26 July 2019 |
| | | | | KR | 10-2298412 | B1 | 06 September 2021 |
| | | | | WO | 2019-132169 | A1 | 04 July 2019 |
| KR | 10-2022-0158737 | A | 01 December 2022 | EP | 4125691 | A1 | 08 February 2023 |
| | | | | JP | 2023-519880 | A | 15 May 2023 |
| | | | | US | 2021-0298846 | A1 | 30 September 2021 |
| | | | | WO | 2021-195369 | A1 | 30 September 2021 |

Form PCT/ISA/210 (patent family annex) (July 2022)